(19) Europäisches Patentamt
European Patent Office
Office européen des brevets

(11) Veröffentlichungsnummer: **0 146 735**
**A1**

(12) **EUROPÄISCHE PATENTANMELDUNG**

(21) Anmeldenummer: **84113272.3**

(22) Anmeldetag: **05.11.84**

(51) Int. Cl.⁴: **C 07 D 405/04**
**C 07 F 7/10**

(30) Priorität: **23.11.83 CH 6286/83**

(43) Veröffentlichungstag der Anmeldung:
**03.07.85 Patentblatt 85/27**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Götschi, Erwin, Dr.**
**Tulpenweg 11**
**CH-4153 Reinach(CH)**

(72) Erfinder: **Hubschwerlen, Christian, Dr.**
**rue de la Gendarmerie**
**F-68200 Durmenach(FR)**

(74) Vertreter: **Martin, Björn et al,**
**Grenzacherstrasse 124 Postfach 3255**
**CH-4002 Basel(CH)**

(54) **Optisch einheitliche beta-Lactame.**

(57) Die neuen Verbindungen der allgemeinen Formel

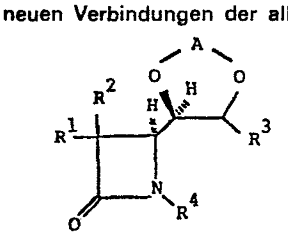

I

worin entweder $R^1$ wasserstoff, einen durch Reduktion leicht entfernbaren Rest, niederes 1-Hydroxyalkyl oder niederes Alkanoyl und $R^2$ Wasserstoff oder $R^1$ und $R^2$ je einen durch Reduktion leicht entfernbaren Rest, $R^3$ Wasserstoff oder niederes Alkyl, $R^4$ Wasserstoff oder eine leicht entfernbare Schutzgruppe und A niederes Alkyliden oder $(C_{5-7})$-Cycloalkyliden bedeuten,
sind wertvolle Zwischenprodukte zur Herstellung von antimikrobiell wirksamen β-Lactamen.

EP 0 146 735 A1

Croydon Printing Company Ltd

F.HOFFMANN-LA ROCHE & CO. Aktiengesellschaft, Basel/Schweiz

RAN 4410/181



# Optisch einheitliche β-Lactame

Die vorliegende Erfindung betrifft optisch einheitliche β-Lactame der allgemeinen Formel

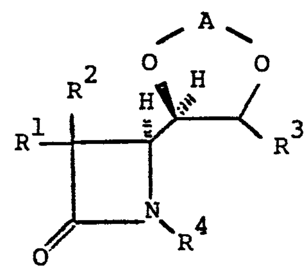

I

worin entweder $R^1$ Wasserstoff, einen durch Reduktion leicht entfernbaren Rest, niederes 1-Hydroxyalkyl oder niederes Alkanoyl und $R^2$ Wasserstoff oder $R^1$ und $R^2$ je einen durch Reduktion leicht entfernbaren Rest, $R^3$ Wasserstoff oder niederes Alkyl, $R^4$ Wasserstoff oder eine leicht entfernbare Schutzgruppe und A niederes Alkyliden oder $(C_{5-7})$-Cycloalkyliden bedeuten.

Der in der vorliegenden Beschreibung verwendete Ausdruck "nieder" bezeichnet Reste und Verbindungen mit höchstens 4 Kohlenstoffatomen. Der Ausdruck "Alkyliden" bezeichnet geradkettige oder verzweigte Kohlenwasserstoffreste mit 2 freien Valenzen am selben Kohlenstoffatom, wie Isopropyliden. Der Ausdruck "$(C_{5-7})$-Cycloalkyliden" be-

Nt/29.8.84

zeichnet cyclische Kohlenwasserstoffreste mit 5-7 Kohlenstoffatomen und 2 freien Valenzen am selben Kohlenstoffatom, wie Cyclohexyliden.

Durch Reduktion leicht entfernbare Reste sind in erster Linie die Halogene Chlor, Brom und Jod, niedere Alkylthiogruppen, niedere Alkylsulfonyloxygruppen, wie Methylsulfonyloxy, und Arylsulfonyloxygruppen, d.h. gegebenenfalls durch niederes Alkyl, Halogen und dergleichen substituierte Phenylsulfonyloxygruppen, wie Benzolsulfonyloxy und p-Toluolsulfonyloxy. Bevorzugte, durch Reduktion leicht entfernbare Reste sind die vorerwähnten Halogene, insbesondere Chlor. Der Ausdruck "leicht entfernbare Schutzgruppe" bezeichnet in erster Linie gegebenenfalls am Phenylring durch niederes Alkoxy substituierte Phenyl- oder 1-Phenyl-niedere Alkylgruppen, niedere 2-Alkenylgruppen, Tri(niedere Alkyl)silylgruppen und dergleichen.

Bevorzugte Verbindungen der Formel I sind diejenigen, worin $R^1$ Wasserstoff oder 1-Hydroxyäthyl und $R^2$ Wasserstoff bedeuten. $R^3$ bedeutet vorzugsweise Wasserstoff, und A bedeutet vorzugsweise niederes Alkyliden. $R^4$ bedeutet vorzugsweise Wasserstoff, 2,4-Dimethoxybenzyl, 3,4-Dimethoxybenzyl, 4-Methoxybenzyl oder t-Butyldimethylsilyl.

Im Rahmen der vorliegenden Erfindung besonders bevorzugte Verbindungen der Formel I sind:

(3S,4S)-1-(2,4-Dimethoxybenzyl)-4-[(R)-2,2-dimethyl-1,3-dioxolan-4-yl]-3-[(R)-1-hydroxyäthyl]-2-azetidinon,

(3S,4S)-1-(t-Butyldimethylsilyl)-4-[(R)-2,2-dimethyl-1,3-dioxolan-4-yl]-3-[(R)-1-hydroxyäthyl]-2-azetidinon,

(S)-1-(2,4-Dimethoxybenzyl)-4-[(R)-2,2-dimethyl-1,3-dioxolan-4-yl]-2-azetidinon,

(S)-1-(t-Butyldimethylsilyl)-4-[(R)-2,2-dimethyl-1,3-dioxolan-4-yl]-2-azetidinon und

(S)-4-[(R)-2,2-Dimethyl-1,3-dioxolan-4-yl]-2-azetidinon.

Die Verbindungen der Formel I können erfindungsgemäss dadurch hergestellt werden, dass man ein reaktives Derivat einer Carbonsäure der allgemeinen Formel

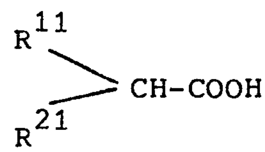

II

worin $R^{11}$ einen durch Reduktion leicht entfernbaren Rest und $R^{21}$ Wasserstoff oder einen durch Reduktion leicht entfernbaren Rest bedeuten,

in Gegenwart einer Base mit einer Verbindung der allgemeinen Formel

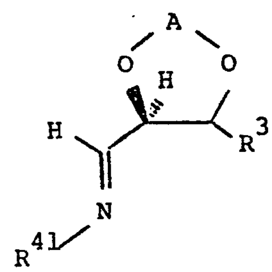

III

worin $R^{41}$ eine leicht entfernbare Schutzgruppe bedeutet und $R^3$ und A die oben angegebene Bedeutung besitzen,

umsetzt, erwünschtenfalls eine erhaltene Verbindung der Formel I, worin $R^1$ einen durch Reduktion leicht entfernbaren Rest, $R^2$ Wasserstoff oder einen durch Reduktion leicht entfernbaren Rest und $R^4$ eine leicht entfernbare Schutzgruppe bedeuten und $R^3$ und A die oben angegebene Bedeutung besitzen, reduziert und vorgängig oder anschliessend die Schutzgruppe entfernt, oder eine erhaltene Verbindung der Formel I, worin $R^1$ und $R^2$ Wasserstoff bedeuten, und $R^3$, $R^4$ und A die oben angegebene Bedeutung besitzen, in Gegenwart einer starken Base mit einem niederen Aldehyd oder einem reaktiven Derivat einer niederen Fettsäure umsetzt, wobei man gegebenenfalls, wenn $R^4$ Wasserstoff bedeutet, das Lactamstickstoffatom vorgängig mit einer leicht entfernbaren Schutzgruppe blockiert, und anschliessend die Schutzgruppe entfernt, oder in einer erhaltenen Verbindung

der Formel I, worin $R^1$ niederes Alkanoyl und $R^2$ Wasserstoff bedeuten, und $R^3$, $R^4$ und A die oben angegebene Bedeutung besitzen, die niedere Alkanoylgruppe reduziert und anschliessend eine allfällig vorhandene Schutzgruppe entfernt.

Bei der Reaktion eines reaktiven Derivates einer Carbonsäure der Formel II mit einer Verbindung der Formel III handelt es sich um eine dem Fachmann geläufige Cyclokondensation. Geeignete reaktive Carbonsäurederivate von Verbindungen der Formel II, worin $R^{11}$ einen durch Reduktion leicht entfernbaren Rest und $R^{21}$ Wasserstoff oder einen durch Reduktion leicht entfernbaren Rest bedeuten, sind beispielsweise die entsprechenden Carbonsäurehalogenide, insbesondere die Carbonsäurechloride, entsprechende Carbonsäureanhydride und gemischte Anhydride (z.B. mit Trifluoressigsäure, aromatischen Sulfonsäuren und dergleichen), entsprechende Carbonsäureimidazolide und dergleichen. Zweckmässigerweise arbeitet man dabei in Gegenwart einer Base, beispielsweise eines tertiären Amins, wie Triäthylamin, und in einem inerten organischen Lösungsmittel, wobei insbesondere Aether, wie Tetrahydrofuran, Diäthyläther, t-Butylmethyläther, Dioxan, Aethylenglykoldimethyläther oder dergleichen, halogenierte Kohlenwasserstoffe, wie Methylenchlorid, Chloroform, 1,2-Dichloräthan oder dergleichen, Toluol, Acetonitril, Dimethylformamid oder dergleichen in Frage kommen. Die obige Cyclokondensation wird dabei in einem Temperaturbereich von etwa -78°C bis etwa 80°C durchgeführt.

Die obige Reaktion einer Verbindung der Formel II mit der optisch einheitlichen Verbindung der Formel III liefert eine entsprechend substituierte Verbindung der obigen Formel I mit der in der allgemeinen Formel I gezeigten Stereochemie. Es hat sich gezeigt, dass die Verwendung der optisch aktiven Verbindung der Formel III in der obigen Cyclokondensation die gewünschte Verbindung in hoher optischer Ausbeute liefert.

Die Reduktion einer erhaltenen Verbindung der Formel I, worin $R^1$ einen durch Reduktion leicht entfernbaren Rest und $R^2$ Wasserstoff oder einen durch Reduktion leicht entfernbaren Rest bedeuten, zu einer Verbindung der Formel I, worin $R^1$ und $R^2$ je Wasserstoff bedeuten, kann nach an sich bekannten und jedem Fachmann geläufigen Methoden erfolgen. Eine für die reduktive Entfernung von Halogenatomen besonders geeignete Methode ist die katalytische Hydrierung in Gegenwart von Katalysatoren, wie Palladium/Kohle oder Raney-Nickel, und in Gegenwart eines säurebindenden Mittels, wie Kaliumhydroxid. Als Lösungsmittel verwendet man dabei vorzugsweise einen niederen Alkohol, wie Methanol. Je nach Reaktivität der eingesetzten Verbindung arbeitet man bei einem Wasserstoffdruck von Normaldruck bis etwa 100 bar und in einem Temperaturbereich von 0°C bis 100°C.

Bei der Reaktion einer Verbindung der Formel I, worin $R^1$ und $R^2$ Wasserstoff bedeuten, mit einem niederen Aldehyd oder einem reaktiven Derivat einer niederen Fettsäure in Gegenwart einer starken Base erhält man eine Verbindung der Formel I, worin $R^1$ niederes 1-Hydroxyalkyl bzw. niederes Alkanoyl und $R^2$ Wasserstoff bedeuten. Es handelt sich dabei ebenfalls um eine dem Fachmann geläufige Umsetzung. Man kann beispielsweise eine Verbindung der Formel I, worin $R^1$, $R^2$ und $R^4$ Wasserstoff bedeuten, mit einem Alkyllithium, wie Butyllithium, in das entsprechende Dilithiumsalz überführen und dieses mit Acetaldehyd oder einem reaktiven Essigsäurederivat, wie Methylacetat, behandeln. Diese Umsetzung wird vorzugsweise unter Schutzgas, z.B. Stickstoff oder Argon, in einem inerten Lösungsmittel, wie Tetrahydrofuran, und bei einer Temperatur zwischen -80° und 50°C durchgeführt. Andererseits kann man auch eine Verbindung der Formel I, worin $R^1$ und $R^2$ Wasserstoff und $R^4$ eine leicht entfernbare Schutzgruppe bedeuten, mit einem Lithiumamid, wie Lithiumdiisopropylamid, in das entsprechende Monolithiumsalz überführen und dieses unter Schutzgas, in einem inerten Lösungsmittel, wie Tetrahydrofuran, und bei einer Temperatur von -80° bis -50°C mit

Acetaldehyd oder einem reaktiven Essigsäurederivat, wie Methylacetat behandeln. Schliesslich kann man auch eine Verbindung der Formel I, worin $R^1$, $R^2$ und $R^4$ Wasserstoff bedeuten, zuerst am Lactamstickstoffatom durch Einführen einer leicht entfernbaren Schutzgruppe blockieren und dann wie vorstehend beschrieben 1-hydroxyalkylieren bzw. alkanoylieren. Die Einführung einer Schutzgruppe erfolgt dabei in an sich bekannter Weise, beispielsweise durch Umsetzen einer entsprechenden Verbindung der Formel I mit einem gegebenenfalls am Phenylring substituierten 1-Phenyl-niederen Alkylhalogenid, 1-Phenyl-niederen Alkyl-niederen Alkylsulfonat oder 1-Phenyl-niederen Alkyl-Arylsulfonat oder einem Tri(niederen Alkyl)halogensilan in einem inerten Lösungsmittel, wie Dimethylformamid, und in Gegenwart eines säurebindenden Mittels, z.B. eines tertiären Amins, wie Triäthylamin. Die Reaktionstemperatur liegt zweckmässigerweise in einem Bereich von etwa 0°C bis Raumtemperatur.

Die Reduktion einer Verbindung der Formel I, worin $R^1$ niederes Alkanoyl und $R^2$ Wasserstoff bedeuten, zu einer Verbindung der Formel I, worin $R^1$ niederes 1-Hydroxyalkyl und $R^2$ Wasserstoff bedeuten, kann ebenfalls nach an sich bekannten und jedem Fachmann geläufigen Methoden erfolgen. Geeignete Reduktionsmittel sind beispielsweise Metallborhydride, wie Natriumborhydrid und Kalium-tris-sec.-butylborhydrid, wobei man die bei solchen Reduktionen üblichen Lösungsmittel und Reaktionsbedingungen verwendet. Die Reduktion kann jedoch auch mittels katalytischer Hydrierung an Platinkatalysatoren bei Raumtemperatur und Normaldruck in einem Lösungsmittel, wie Aethylacetat oder Methanol, bewerkstelligt werden.

Schliesslich kann auch die Entfernung der in Verbindungen der Formel I allfällig vorhandenen N-Schutzgruppen nach an sich bekannten und jedem Fachmann geläufigen Methoden erfolgen. Die 2,4-Dimethoxybenzylgruppe kann beispielsweise durch Behandeln einer entsprechend geschützten Verbindung der Formel I mit Kaliumpersulfat in wässrigem Acetonitril bei erhöhter Temperatur entfernt werden.

Die Verbindungen der Formel I sind wertvolle Zwischenprodukte zur Herstellung von antimikrobiell wirksamen β-Lactamen. Sie können beispielsweise gemäss dem nachfolgenden Reaktionsschema in (+)-Thienamycin übergeführt werden:

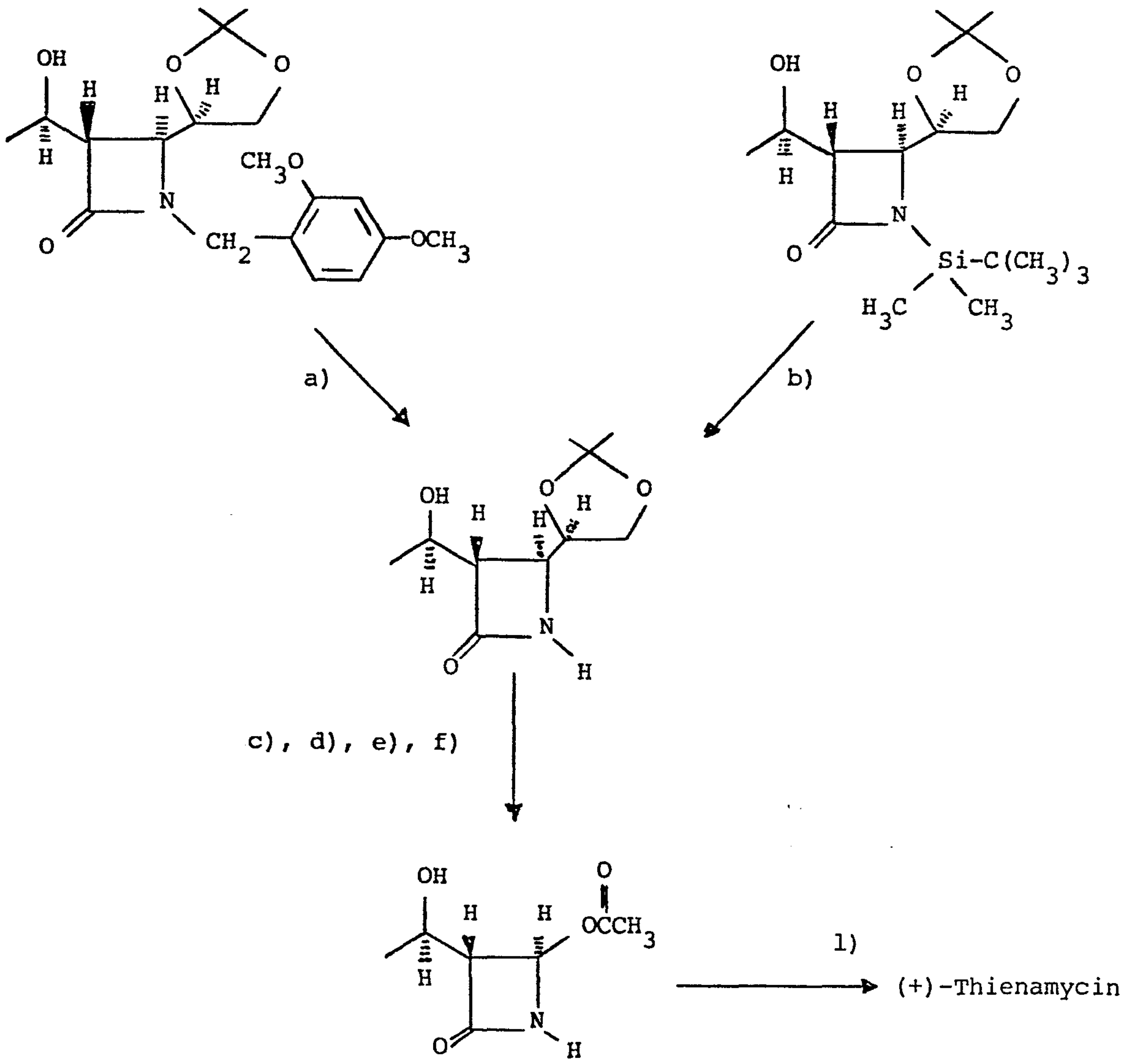


a) $K_2S_2O_8/CH_3CN/H_2O$; b) $NH_4F$/MeOH; c) p-TsOH/MeOH/$H_2O$; d) $NaJO_4$/MeOH/$H_2O$; e) $Ag_2O/H_2O$; f) $Pb(OAc)_4$/DMF/HOAc.

1) S. Karady, J.S. Amato, R.A. Reamer, L.M. Weinstock, J. Amer.Chem. Soc. 103, 6765 (1981).

Die nachfolgenden Beispiele dienen der näheren Erläuterung der vorliegenden Erfindung. Alle Temperaturen sind in Celsiusgraden angegeben.

## Beispiel 1

a) Zu einer bei Raumtemperatur gerührten Lösung von 12,3 g (95,0 mMol) 2,3-O-Isopropyliden-L-glyceraldehyd in 100 ml trockenem Methylenchlorid (frei von Methanol) gibt man 10 g Molekularsieb 4Å und anschliessend tropfenweise eine Lösung von 15,86 g (95 mMol) 2,4-Dimethoxybenzylamin in 20 ml trockenem Methylenchlorid. Man rührt die Reaktionsmischung während 2 Stunden bei Raumtemperatur, versetzt anschliessend mit 5 g wasserfreiem Magnesiumsulfat, rührt noch während 30 Minuten und filtriert anschliessend, wobei der Filterkuchen noch mit 20 ml Methylenchlorid gewaschen wird.

b) Die erhaltene organische Lösung von Isopropyliden-L-glyceraldehyd-(2,4-dimethoxybenzyl)imin wird unter Argon auf -5° gekühlt und unter Rühren mit 17,2 ml (95 mMol) Triäthylamin versetzt. Nach wenigen Minuten gibt man über einen Zeitraum von 1 Stunde eine Lösung von 7,9 ml (73,4 mMol) Chloracetylchlorid in 100 ml trockenem Methylenchlorid dazu, rührt die Reaktionsmischung während 2 Stunden bei -5° und lässt anschliessend auf Raumtemperatur erwärmen. Man rührt noch über Nacht bei Raumtemperatur, wäscht die Reaktionsmischung dreimal mit je 100 ml Wasser und mit 100 ml Natriumchloridlösung und trocknet die erhaltene Lösung über Natriumsulfat. Man dampft ein und kristallisiert den Rückstand aus Aethylacetat. Nach Umkristallisieren aus Aethylacetat erhält man (3S,4R)-3-Chlor-1-(2,4-dimethoxybenzyl)-4-[(R)-2,2-dimethyl-1,3-dioxolan-4-yl]-2-azetidinon als farblose Kristalle vom Schmelzpunkt 140-142°; $[\alpha]_D^{20}$ = +33,2° (c = 1, Chloroform).

## Beispiel 2

Eine Lösung von 4,26 g (12,0 mMol) (3S,4R)-3-Chlor-1-(2,4-dimethoxybenzyl)-4-[(R)-2,2-dimethyl-1,3-dioxolan-4-yl]-2-azetidinon und 3 g (53 mMol) Kaliumhydroxyd in 50 ml Methanol wird in der Gegenwart von 400 mg 5-proz.

Palladium/Kohle während 1 Stunde bei Normaldruck hydriert. Man filtriert vom Katalysator ab, verdünnt mit Aethylacetat und wäscht mit 1M Phosphatpuffer (pH 7) und gesättigter Natriumchloridlösung. Die organische Phase wird über Natriumsulfat getrocknet und eingedampft, worauf der Rückstand an Kieselgel mit Aethylacetat/Hexan als Eluens chromatographiert wird. Man erhält nach zweimaligem Kristallisieren aus Methylenchlorid/Hexan (S)-1-(2,4-Dimethoxybenzyl)-4-[(R)-2,2-dimethyl-1,3-dioxolan-4-yl]-2-azetidinon als weisse Kristalle vom Schmelzpunkt 85-86°; $[\alpha]_D^{20}$ = +50,3° (c = 1, Chloroform).

## Beispiel 3

Zu einer auf -65° gekühlten Lösung von 111 mg (1,1 mMol) Diisopropylamin in 4 ml Tetrahydrofuran gibt man 0,65 ml einer 1,7M Lösung von Butyllithium in Hexan. Man kühlt auf -78° und versetzt nach 15 Minuten mit einer Lösung von 321 mg (1 mMol) (S)-1-(2,4-Dimethoxybenzyl)-4-[(R)-2,2-dimethyl-1,3-dioxolan-4-yl]-2-azetidinon in 1 ml Tetrahydrofuran. Man rührt während 45 Minuten bei -78° und tropft dann innert 3 Minuten eine Lösung von 100 mg (   mMol) Acetaldehyd in 0,5 ml Tetrahydrofuran zu, wobei die Temperatur auf -70° ansteigt. Man rührt während 10 Minuten, entfernt das Kühlbad, versetzt die Reaktionslösung mit 2 ml 14-proz. wässriger Ammoniumchloridlösung und extrahiert mit 30 ml Aethylacetat. Die organische Phase wird mit gesättigter Natriumchloridlösung gewaschen, über Natriumsulfat getrocknet und im Vakuum eingedampft. Der Rückstand wird an 10 g Kieselgel mit Aethylacetat/Hexan (2:1) als Eluens chromatographiert. Man erhält (3S,4S)-1-(2,4-Dimethoxybenzyl)-4-[(R)-2,2-dimethyl-1,3-dioxolan-4-yl]-3-[(R)-1-hydroxyäthyl]-2-azetidinon, das nach Kristallisieren aus Aethylacetat/Hexan als feine Nadeln vom Schmelzpunkt 145-146° anfällt.

## Beispiel 4

Zu einer auf 80° erwärmten Suspension von 0,964 g (3 mMol) (S)-1-(2,4-Dimethoxybenzyl)-4-[(R)-2,2-dimethyl-1,3-dioxolan-4-yl]-2-azetidinon in einem Gemisch von 10 ml Wasser und 10 ml Acetonitril tropft man innert 30 Minuten eine Lösung von 1,47 g (5,45 mMol) Kaliumpersulfat in 25 ml Wasser, wobei der pH des Reaktionsgemisches durch Zugabe von gesättigter Natriumhydrogencarbonatlösung auf 5 gehalten wird. Man rührt noch während 1 Stunde bei pH 5, kühlt dann ab und extrahiert das Reaktionsgemisch mit dreimal je 50 ml Methylenchlorid. Die organischen Phasen werden mit 20 ml gesättigter Natriumchloridlösung gewaschen, über Natriumsulfat getrocknet und eingedampft. Der Rückstand wird an Kieselgel mit Aethylacetat/Hexan (3:1) als Eluens chromatographiert. Durch Kristallisieren des erhaltenen Materials aus Aethylacetat/Hexan erhält man (S)-4-[(R)-2,2-Dimethyl-1,3-dioxolan-4-yl]-2-azetidinon als weisse Kristalle vom Schmelzpunkt 91-92°; $[\alpha]_D^{20}$ = +13,6° (c = 1, Aethylacetat).

## Beispiel 5

Man versetzt eine auf 0° gekühlte Lösung von 17,1 g (0,1 Mol) (S)-4-[(R)-2,2-Dimethyl-1,3-dioxolan-4-yl]-2-azetidinon und 15,1 g (0,1 Mol) t-Butyldimethylchlorsilan in 100 ml Dimethylformamid unter Rühren mit 10,6 g (0,105 Mol) Triäthylamin, wobei sofort ein Niederschlag entsteht. Man rührt während 2 Stunden bei 0°, verdünnt dann mit 200 ml Aether und wäscht das Reaktionsgemisch fünfmal mit je 80 ml Wasser. Die Wasserphasen werden dann noch mit 100 ml Aether extrahiert. Die organischen Phasen werden über Natriumsulfat getrocknet und eingedampft, worauf man das verbleibende Oel destilliert. Man erhält (S)-1-(t-Butyldimethylsilyl)-4-[(R)-2,2-dimethyl-1,3-dioxolan-4-yl]-2-azetidinon vom Siedepunkt 84-87° (0,5 Torr); $[\alpha]_D^{20}$ = -22,7° (c = 1, Aethylacetat).

Beispiel 6

Zu einer auf -65° gekühlten Lösung von 2,78 g (27,5 mMol) Diisopropylamin in 100 ml Tetrahydrofuran tropft man innert 5 Minuten 16,2 ml einer 1,7M-Lösung von Butyllithium in Hexan. Man kühlt dann auf -76° ab und versetzt nach 15 Minuten mit einer Lösung von 7,14 g (25 mMol) (S)-1-(t-Butyldimethylsilyl)-4-[(R)-2,2-dimethyl-1,3-dioxolan-4-yl]-2-azetidinon in 6 ml Tetrahydrofuran. Man rührt während 30 Minuten bei -76° und tropft dann innert 10 Minuten eine Lösung von 2,65 g Acetaldehyd in 5 ml Tetrahydrofuran zu. Man rührt während 15 Minuten, entfernt das Kühlbad, versetzt die Reaktionslösung mit 20 ml 14-proz. wässriger Ammoniumchloridlösung und extrahiert mit 100 ml Aethylacetat. Die organische Phase wird mit gesättigter Natriumchloridlösung gewaschen, über Natriumsulfat getrocknet und im Vakuum eingedampft. Das verbleibende Oel wird in 10 ml Hexan aufgenommen und die Lösung wird bei -20° stehen gelassen. Man erhält farblose Kristalle vom Schmelzpunkt 80-90°. Nach wiederholtem Umkristallisieren aus Aethylacetat/Hexan erhält man (3S,4S)-1-(t-Butyldimethylsilyl)-4-[(R)-2,2-dimethyl-1,3-dioxolan-4-yl]-3-[(R)-1-hydroxyäthyl]-2-azetidinon vom Schmelzpunkt 95-96°; $[\alpha]_D^{20}$ = -16,6° (c = 1, Aethylacetat).

## Patentansprüche

1. Verbindungen der allgemeinen Formel

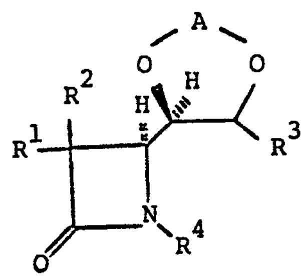

I

worin entweder $R^1$ Wasserstoff, einen durch Reduktion leicht entfernbaren Rest, niederes 1-Hydroxyalkyl oder niederes Alkanoyl und $R^2$ Wasserstoff oder $R^1$ und $R^2$ je einen durch Reduktion leicht entfernbaren Rest, $R^3$ Wasserstoff oder niederes Alkyl, $R^4$ Wasserstoff oder eine leicht entfernbare Schutzgruppe und A niederes Alkyliden oder $(C_{5-7})$-Cycloalkyliden bedeuten.

2. Verbindungen gemäss Anspruch 1, worin $R^1$ Wasserstoff oder 1-Hydroxyäthyl und $R^2$ Wasserstoff bedeuten.

3. Verbindungen gemäss Anspruch 1 oder 2, worin A niederes Alkyliden und $R^3$ Wasserstoff bedeuten.

4. Verbindungen gemäss einem der Ansprüche 1-3, worin $R^4$ Wasserstoff, 2,4-Dimethoxybenzyl, 3,4-Dimethoxybenzyl, 4-Methoxybenzyl oder t-Butyldimethylsilyl bedeutet.

5. (3S,4S)-1-(2,4-Dimethoxybenzyl)-4-[(R)-2,2-dimethyl-1,3-dioxolan-4-yl]-3-[(R)-1-hydroxyäthyl]-2-azetidinon.

6. (3S,4S)-1-(t-Butyldimethylsilyl)-4-[(R)-2,2-dimethyl-1,3-dioxolan-4-yl]-3-[(R)-1-hydroxyäthyl]-2-azetidinon.

7. (S)-1-(2,4-Dimethoxybenzyl)-4-[(R)-2,2-dimethyl-1,3-dioxolan-4-yl]-2-azetidinon.

8. (S)-1-(t-Butyldimethylsilyl)-4-[(R)-2,2-dimethyl-1,3-dioxolan-4-yl]-2-azetidinon.

9. (S)-4-[(R)-2,2-Dimethyl-1,3-dioxolan-4-yl]-2-azetidinon.

10. Verfahren zur Herstellung von Verbindungen gemäss einem der Ansprüche 1-9, dadurch gekennzeichnet, dass man ein reaktives Derivat einer Carbonsäure der allgemeinen Formel

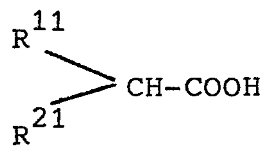

II

worin $R^{11}$ einen durch Reduktion leicht entfernbaren Rest und $R^{21}$ Wasserstoff oder einen durch Reduktion leicht entfernbaren Rest bedeuten,

in Gegenwart einer Base mit einer Verbindung der allgemeinen Formel

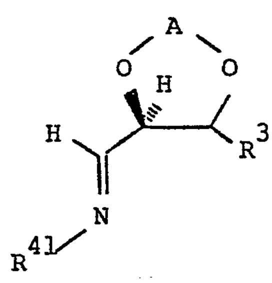

III

worin $R^{41}$ eine leicht entfernbare Schutzgruppe bedeutet, und $R^3$ und A die in Anspruch 1 angegebene Bedeutung besitzen,

umsetzt, erwünschtenfalls eine erhaltene Verbindung der Formel I, worin $R^1$ einen durch Reduktion leicht entfernbaren Rest, $R^2$ Wasserstoff oder einen durch Reduktion leicht entfernbaren Rest und $R^4$ eine leicht entfernbare Schutzgruppe bedeuten und $R^3$ und A die in Anspruch 1 angegebene Bedeutung besitzen, reduziert und vorgängig oder anschliessend die Schutzgruppe entfernt, oder eine erhaltene Verbindung der Formel I, worin $R^1$ und $R^2$ Wasserstoff be-

deuten, und $R^3$, $R^4$ und A die in Anspruch 1 angegebene Bedeutung besitzen, in Gegenwart einer starken Base mit einem niederen Aldehyd oder einem reaktiven Derivat einer niederen Fettsäure umsetzt, wobei man gegebenenfalls, wenn $R^4$ Wasserstoff bedeutet, das Lactamstickstoffatom vorgängig mit einer leicht entfernbaren Schutzgruppe blockiert, und anschliessend die Schutzgruppe entfernt, oder in einer erhaltenen Verbindung der Formel I, worin $R^1$ niederes Alkanoyl und $R^2$ Wasserstoff bedeuten, und $R^3$, $R^4$ und A die in Anspruch 1 angegebene Bedeutung besitzen, die niedere Alkanoylgruppe reduziert und anschliessend eine allfällig vorhandene Schutzgruppe entfernt.

11. Verwendung von Verbindungen gemäss einem der Ansprüche 1-9 bei der Herstellung von antimikrobiell wirksamen β-Lactamen.

***

Patentansprüche für Oesterreich

1. Verfahren zur Herstellung von Verbindungen der allgemeinen Formel

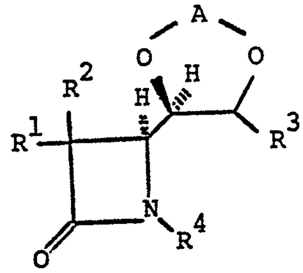

I

worin entweder $R^1$ Wasserstoff, einen durch Reduktion leicht entfernbaren Rest, niederes 1-Hydroxyalkyl oder niederes Alkanoyl und $R^2$ Wasserstoff oder $R^1$ und $R^2$ je einen durch Reduktion leicht entfernbaren Rest, $R^3$ Wasserstoff oder niederes Alkyl, $R^4$ Wasserstoff oder eine leicht entfernbare Schutzgruppe und A niederes Alkyliden oder $(C_{5-7})$-Cycloalkyliden bedeuten,

dadurch gekennzeichnet, dass man ein reaktives Derivat einer Carbonsäure der allgemeinen Formel

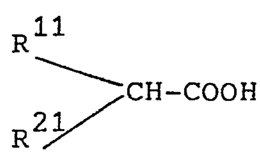

II

worin $R^{11}$ einen durch Reduktion leicht entfernbaren Rest und $R^{21}$ Wasserstoff oder einen durch Reduktion leicht entfernbaren Rest bedeuten,

in Gegenwart einer Base mit einer Verbindung der allgemeinen Formel

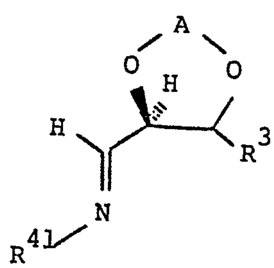

III

worin $R^{41}$ eine leicht entfernbare Schutzgruppe be-

deutet, und $R^3$ und A die in Anspruch 1 angegebene Bedeutung besitzen,

umsetzt, erwünschtenfalls eine erhaltene Verbindung der Formel I, worin $R^1$ einen durch Reduktion leicht entfernbaren Rest, $R^2$ Wasserstoff oder einen durch Reduktion leicht entfernbaren Rest und $R^4$ eine leicht entfernbare Schutzgruppe bedeuten und $R^3$ und A die in Anspruch 1 angegebene Bedeutung besitzen, reduziert und vorgängig oder anschliessend die Schutzgruppe entfernt, oder eine erhaltene Verbindung der Formel I, worin $R^1$ und $R^2$ Wasserstoff bedeuten, und $R^3$, $R^4$ und A die in Anspruch 1 angegebene Bedeutung besitzen, in Gegenwart einer starken Base mit einem niederen Aldehyd oder einem reaktiven Derivat einer niederen Fettsäure umsetzt, wobei man gegebenenfalls, wenn $R^4$ Wasserstoff bedeutet, das Lactamstickstoffatom vorgängig mit einer leicht entfernbaren Schutzgruppe blockiert, und anschliessend die Schutzgruppe entfernt, oder in einer erhaltenen Verbindung der Formel I, worin $R^1$ niederes Alkanoyl und $R^2$ Wasserstoff bedeuten, und $R^3$, $R^4$ und A die in Anspruch 1 angegebene Bedeutung besitzen, die niedere Alkanoylgruppe reduziert und anschliessend eine allfällig vorhandene Schutzgruppe entfernt.

2. Verfahren gemäss Anspruch 1, dadurch gekennzeichnet, dass man Verbindungen der Formel I herstellt, worin $R^1$ Wasserstoff oder 1-Hydroxyäthyl und $R^2$ Wasserstoff bedeuten.

3. Verfahren gemäss Anspruch 1 oder 2, dadurch gekennzeichnet, dass man Verbindungen der Formel I herstellt, worin A niederes Alkyliden und $R^3$ Wasserstoff bedeuten.

4. Verfahren nach einem der Ansprüche 1-3, dadurch gekennzeichnet, dass man Verbindungen der Formel I herstellt, worin $R^4$ Wasserstoff, 2,4-Dimethoxybenzyl, 3,4-Dimethoxybenzyl, 4-Methoxybenzyl oder t-Butyldimethylsilyl bedeutet.

5. Verfahren gemäss Anspruch 1, dadurch gekennzeichnet, dass man (3S,4S)-1-(2,4-Dimethoxybenzyl)-4-[(R)-2,2-dimethyl-1,3-dioxolan-4-yl]-3-[(R)-1-hydroxyäthyl]-2-azetidinon herstellt.

6. Verfahren gemäss Anspruch 1, dadurch gekennzeichnet, dass man (3S,4S)-1-(t-Butyldimethylsilyl)-4-[(R)-2,2-dimethyl-1,3-dioxolan-4-yl]-3-[(R)-1-hydroxyäthyl]-2-azetidinon herstellt.

7. Verfahren gemäss Anspruch 1, dadurch gekennzeichnet, dass man (S)-1-(2,4-Dimethoxybenzyl)-4-[(R)-2,2-dimethyl-1,3-dioxolan-4-yl]-2-azetidinon herstellt.

8. Verfahren gemäss Anspruch 1, dadurch gekennzeichnet, dass man (S)-1-(t-Butyldimethylsilyl)-4-[(R)-2,2-dimethyl-1,3-dioxolan-4-yl]-2-azetidinon herstellt.

9. Verfahren gemäss Anspruch 1, dadurch gekennzeichnet, dass man (S)-4-[(R)-2,2-Dimethyl-1,3-dioxolan- 4-yl]-2-azetidinon herstellt.

10. Verwendung von Verbindungen der Formel I bei der Herstellung von antimikrobiell wirksamen β-Lactamen.

***

Europäisches Patentamt

# EUROPÄISCHER RECHERCHENBERICHT

0146735
Nummer der Anmeldung

EP 84 11 3272

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | TETRAHEDRON LETTERS, Band 24, Nr. 29, Seiten 3009-3012, 1983 Pergamon Press Ltd. OXFORD, (GB)<br>* Insgesamt, (siehe u.a. Formel 5, Seite 3010) * | 1-4, 9-11 | C 07 D 405/04<br>C 07 F 7/10 |
| A | JOURNAL OF THE CHEMICAL SOCIETY. PERKIN TRANSACTIONS I, Band 3, 1983, Royal society of chemistry, Seiten 605-611. R.M. ADLINGTON et al.:"Synthesis of 3-Methyleneazetidin-2-one derivatives from alpha-keto-amides".<br>* Insgesamt, (siehe u.a. Seite 606, Formel 5; Seite 609, Spalte 2, Absatz 3) * | 1,10, 11 | |
| | | | RECHERCHIERTE SACHGEBIETE (Int. Cl.4) |
| | | | C 07 D 405/00<br>C 07 D 205/00<br>C 07 F 7/00<br>C 07 D 487/00 |
| A | EP-A-0 073 061 (F. HOFFMANN-LA ROCHE & CO.)<br>* Ansprüche * | 1,10, 11 | |
| A | ORGANIC CHEMISTRY, Band 47, Nr. 12, June 4, 1982, Seiten 2328-2331 TETSUJI KAMETANI et al.:"Further studies on the synthesis of thienamycin: a facile and stereo-selective synthesis of a bicyclic<br>-/- | 1,11 | |

Der vorliegende Recherchenbericht wurde für alle Patentansprüche erstellt.

| Recherchenort | Abschlußdatum der Recherche | Prufer |
|---|---|---|
| DEN HAAG | 01-02-1985 | CHOULY J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**EUROPÄISCHER RECHERCHENBERICHT**

0146735
Nummer der Anmeldung

EP 84 11 3272

Seite 2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| | beta-keto ester by 1,3-dipolar cycloaddition". <br> * Insgesamt * <br> ----- | | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| DEN HAAG | 01-02-1985 | CHOULY J. |

KATEGORIE DER GENANNTEN DOKUMENTEN
X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82